(19) Europäisches Patentamt
European Patent Office
Office européen des brevets

(11) Publication number: **0 076 721**
**B1**

(12) **EUROPEAN PATENT SPECIFICATION**

(45) Date of publication of patent specification: **06.02.85**

(21) Application number: **82401702.4**

(22) Date of filing: **20.09.82**

(51) Int. Cl.$^4$: **C 07 C 51/10,** C 07 C 57/30, C 07 C 57/58, C 07 C 59/64, C 07 C 59/84, C 07 D 307/54, B 01 J 31/28 // A61K31/195

(54) **Process for the carbonylation of secondary benzyl halides.**

(30) Priority: **21.09.81 IT 2405381**

(43) Date of publication of application:
**13.04.83 Bulletin 83/15**

(45) Publication of the grant of the patent:
**06.02.85 Bulletin 85/06**

(84) Designated Contracting States:
**BE CH DE FR GB LI NL**

(56) References cited:
**CH-A- 589 593**
**DE-A-2 828 041**
**DE-B-2 600 541**
**GB-A- 988 954**
**GB-A-2 079 748**
**US-A-4 102 920**
**US-A-4 102 921**

(73) Proprietor: **Montedison S.p.A.**
***Patents & Licensing Dept. Foro Buonaparte, 31***
**P.O. Box 10528**
**I-20121 Milan (IT)**

(72) Inventor: **Francalanci, Franco**
**7 C.so Torino**
**Novara (IT)**
Inventor: **Foa', Marco**
**19 Via del Sabbione**
**Novara (IT)**
Inventor: **Gardano, Andrea**
**11 C.so Roma**
**Trino Vercellese Vercelli (IT)**

(74) Representative: **Hirsch, Marc-Roger**
**34 rue de Bassano**
**F-75008 Paris (FR)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention concerns a process for the carbonlyation of secondary benzyl halides.

More particularly, the present invention relates to a catalytic process for the synthesis of alkaline salts of alpha-aryl-propionic acids, and of 3-aryl-3-methylpyruvic or alpha-keto-beta-aryl-butyric acids, starting from the corresponding 1-halogen-1-arylethanes, (also referred to the following present description as "benzyl halides"), and from carbon monoxide in a biphasic liquid/liquid system, in the presence of alkaline bases.

More particularly, the above process is carried out in the presence of a catalyst consisting of carbonyl complexes of cobalt.

From the alkaline salts the corresponding acids are easily obtainable by acidification with mineral acids (such as HCl, $H_2SO_4$, etc.), by extraction, etc., according to conventional methods.

The process is based on the carbonylation reaction of 1-halogen-1-arylethanes and involves the insertion of the carboxylic group or of the alpha-keto-carboxylic group on a secondary carbon atom under catalytic conditions provided by Co-carbonyl complexes, in the presence of alkaline bases, said catalysis being carried out in a biphasic organic/aqueous system.

The organic carboxylic acids obtained according to the present invention present the following general formula (I):

$$Ar-\underset{\displaystyle CH_3}{\underset{|}{CH}}-(CO)_nOH \qquad (I)$$

wherein Ar represents either an aromatic or a heteroaromatic group containing one or several rings, linked together, and having overall up to 20 carbon atoms, and preferably being a phenyl, naphthyl, diphenyl, or thienyl group, while n is an integer equal to 1 or 2.

Said Ar group may in turn be substituted by groups that are inert under reaction conditions. Compatible groups are, for instance, alkylic, alicyclic or arylic groups, each of them optionally substituted, halogen, alcoxyl, phenoxyl, ketone groups, etc.

The compounds obtained represent worthwhile products having useful applications in a wide range of commercial fields.

More particularly, the alpha-aryl-propionic acids represent important products in the field of high purity chemicals as well as in the field of phytodrugs and especially of pharmaceutical products.

Products of this class, such as for instance 2-(4'-isobutylphenyl)-propionic acid and 2-(6'-methoxy-2'-naphthyl)-propionic acid, are of special interest to the pharmaceutical industry as antiinflammatory, analgesical, antipyrethical agents, etc.

As stated herein-above, the process of this invention is based on the carbonylation reaction conducted on a secondary benzyl halide having the following formula II:

$$Ar-\underset{\displaystyle CH_3}{\underset{|}{CH}}-X \qquad (II)$$

wherein Ar has already been defined, and X is Cl or Br, in the presence of a catalytic system consisting of carbonylic complexes of Co, in a biphasic organic liquid/aqueous liquid system, more clearly defined herein-below.

Research has only recently turned towards the carbonylation of substrates of the secondary benzyl type, such as for instance the secondary aryl-alkyl halides. This type of carbonylation, besides raising problems as to the reaction mechanism involved, also has the drawback of leading to operational difficulties as to the possible yields, etc.

Literature on the subject is not particularly extensive especially as regards commercial production.

Practically, it may, however, be affirmed that, up to now, the alpha-aryl-propionic acids and alkaline salts thereof, subject of the present invention, have been prepared by different methods such as among others hydrolysis of the corresponding nitriles; reaction of $CO_2$ with Grignard compounds; decarbonylation of malonic derivatives; oxidation of alcohols or of aldehydes having a suitable structure; or by reduction of aryl-acrylic acids, etc.

In fact these methods, technologically distinct from the process of the present invention, are characterized in that they are multi-step, non-catalytic methods, and especially complicated by the use of reactants not easily available and/or manipulable; they consequently have corresponding operational and economical drawbacks that make them of little practical commercial interest.

On the other hand, catalytic methods for the preparation of alpha-aryl-propionic acids have recently been suggested.

According to one of these methods, substituted alpha-aryl-propionic acids or alkyl esters thereof are prepared by means of either hydrocarboxylation or by hydrocarbalkoxylation of substituted aryl-

ethylenes in either alcohol or aqueous mediums, catalyzed by palladium complexes, preferably in the presence of acids.

Nonetheless, the commercial interest offered by the above method does not appear high. In fact this method, among other difficulties involved, foresees the use of expensive Pd complexes as catalysts and the use of high CO pressures.

In the prior art carbonylation reactions of halogen derivatives of the type of formula (II) with Pd and Co complexes are also reported, these reactions producing esters of alpha-aryl-propionic acids.

In the first case the use of expensive catalysts such as the arsinic complexes of Pd under CO pressure is foreseen. Moreover, the esters obtained have poor yields and low selectivity.

In the second case, the method foresees the formation of esters by the use of alkaline alcoholates in the presence of dicobalto-octacarbonyl. Also in this case yields and selectivities are poor; in fact, substantial amounts of linear isomer acid ethers and esters are obtained. This involves operational drawbacks as to the separation and purification of the products.

Moreover, a further economical consists in the use of alcoholates under strictly controlled pH conditions.

Further, the reaction proves strictly limited to phenyl derivatives, possibly only alkyl substituted phenyl derivatives.

On the other hand, methods have been described for the preparation of alpha-keto-beta-aryl-butyric acids, according to formula I in which n=2. These are laboratory methods and, thus, of little commercial interest, not being adapted to the technology according to the present invention.

The object of the present invention is to provide a simple and inexpensive catalytic process for the preparation, on an commercial scale, of the acids of formula (I) or of their alkaline salts, that is free of the drawbacks of the prior art analyzed herein-above.

In fact, the process according to the present invention, provides for the carbonylation of secondary benzyl substrates of formula (II), conducted on a secondary carbon atom under mild operational conditions such as to allow the recycling of the catalytic system consisting of a cobalt hydrocarbonyl salt.

This process has the advantage of offering applicational flexibility that will allow the obtention of a much wider range of alpha-aryl-propionic and keto-butyric acid products as well as of their alkaline salts.

These and still other objects and advantages, which will appear more clearly to those skilled in the Art in the following description, are achieved, according to this invention, by a process for the preparation of acids of formula (I), as previously defined, characterized in that a 1-halo-1-arylethane of formula (II), as previously defined, is made to react with carbon monoxide and with an alkaline hydroxide, at a temperature comprised between about 20°C and 70°C and under a pressure comprised between 1 and 10 atmospheres, in a biphasic aqueous/organic liquid system consisting of:

a) an aqueous phase containing the alkaline hydroxide;

b) an organic phase consisting of the initial halide of formula (II) dissolved in an organic solvent substantially unmixable with the aqueous alkaline phase, in the presence of an "onium" salt, more precisely defined herein-above, and of a catalysts consisting of a cobalt hydrocarbonyl salt or at least one of its precursors.

The reaction may be schematically represented by the following equations:

1) $$Ar-\overset{CH}{\underset{H}{C}}-X+CO+2MOH \xrightarrow[\text{MOH/}H_2O\text{/organic solvent}]{\{Co(CO)_4\}^-\text{/"onium" salt}}$$

(II)

$$\longrightarrow Ar-\overset{CH_3}{\underset{H}{C}}-COOM+MX+H_2O$$

2) $$(II)+2CO+2MOH \xrightarrow[\text{MOH/}H_2O\text{/organic solvent}]{\{Co(CO)_4\}^-\text{/"onium" salt}}$$

$$\longrightarrow Ar-\overset{CH_3}{\underset{H}{C}}-(CO)_2OM+MX+H_2O$$

wherein M represents Na, K or Li, and the organic solvent is further defined herein-below, the other symbols having the meaning stated herein-above.

The organic solvent must be substantially unmixable with the aqueous alkaline phase. Effective solvents proved to be: linear or branched aliphatic or cycloaliphatic alcohols having from 3 to 10 carbon atoms, possibly arylsubstituted, aromatic and aliphatic ethers, aromatic hydrocarbons.

Particularly suitable solvents proved to be: benzene, diphenyl-ether, t-butyl-methyl-ether, n-propyl alcohol, isopropyl alcohol, n-butyl alcohol, sec-butyl alcohol, neopentyl alcohol, n-amyl alcohol, t-amyl alcohol, cyclo-hexanol and 1-phenylethanol.

The catalysts are cobalt hydrocarbonyl salts having the formula (III):

$$Me^{n+}\{Co(CO)_4\}_n \qquad (III)$$

wherein Me represents a cation of a metal with valence n, such as the alkaline metals (Na, K, Li) or cobalt, iron, manganese etc., these cobalt hydrocarbonyl salts being known and prepared according to conventional methods.

Preferred catalysts are sodium, cobalt, manganese and iron salts of formula (III).

"Precursors" of said salts may, however, also be used.

By the term "precursor", used in the present description, is meant one or more compounds which in the reaction conditions form the above specified cobalt hydrocarbonyl salt.

For instance, the cobalt hydrocarbonyl alkaline salt may be obtained *"in situ"* from $Co_2(CO)_8$ under the prevailing reaction conditions.

In another embodiment of the process according to the invention, the cobalt hydrocarbonyl salt catalyst may preferably be prepared, according to this invention, for instance, from a cobalt salt such as a chloride, sulphate, bromide, etc., from an Fe/Mn alloy powder (containing about 80% Mn) and from sulphurated promoters, in either a methyl or ethyl alcohol, under a carbon monoxide pressure comprised between 1 and 2 atmospheres and at a temperature comprised between 10°C and 80°C, but preferably comprised between about 25°C and 35°C.

The concentration of the cobalt salt in the solution is comprised between 0.3 and 1 mol/litre. Per each mole of cobalt salt are used from 1 to 2 moles of Mn in the form of a Fe/Mn alloy. The Fe/Mn alloy is ground beforehand so as to pass through a screen of at least 5000 mesh/sq.cm (thus having openings of 0.290 mm).

The preferred sulphurated promoters are: sodium sulphide and thiosulphate, which are used in quantities comprised between 0.01 and 0.1 mol/mol of cobalt salt.

The alcohol mixture containing the cobalt salt, the alloy and the sulphurated promoter in the alcohol solvent, is kept under vigorous stirring under a CO atmosphere for a time sufficient to complete the absorption of the CO, this time being at least 2—3 hours. In this way Mn and/or Fe salts of cobalt hydrocarbonyl are obtained. The decanted solution may be directly introduced into the reaction while the solvent may be removed under vacuum, thereby obtaining the catalysts of the invention.

In a further embodiment of the process according to the invention, the sodium salt of $Co_2(CO)_8$ may also be obtained by reduction with sodium amalgam in an ether solvent (tetrahydrofuran). $Co_2(CO)_8$ is prepared, for instance, from $CoCO_3$ under a CO and hydrogen pressure in petroleum ether.

The molar ratio between the 1-halogen-1-arylethane (II) and the cobalt hydrocarbonyl salt catalyst may vary within a wide range. Convenient results may be obtained, in any case, with values of said molar ratio comprised between 10:1 and about 150:1.

The reaction temperature is comprised, as previously indicated, between 20°C and 70°C. The carbon monoxide pressure is comprised between about 1 and 10 atmospheres, although for obtaining compounds (I) with n=2, the preferred pressures are equal to or greater than 2 atmospheres.

The reaction is usually completed within a time period of about 1 to 24 hours, depending on parametrical conditions of temperature; on concentration; on the type of secondary benzyl halide (II) and on the catalyst used; etc.

Alkaline hydroxides of Na, K, Li, preferably chosen from among sodium and potassium hydroxides, are also used, their concentration in the aqueous phase being preferably maintained between about 20% and 50% by weight, and in any case well in excess of the stoichiometric proportion with respect to the 1-halogen-1-arylethane.

Effective initial halides of formula (II) proved to be: 1-bromo-1-phenylethane, 1-chloro-1-(6'-methoxy-2'-naphthyl)-ethane, 1-bromo-1-(p.chorophenyl)-ethane, 1-bromo-1-(p.isobutylphenyl)-ethane, 1-bromo-1-(m-chlorophenyl)-ethane, 1-chloro-1-phenylethane. The initial 1-halogen-1-arylethane (II) may be introduced into the reactor either gradually or in one single batch charge at the beginning of the reaction.

The reaction itself is conducted according to the known phase-transfer techniqe, in the presence of an "onium" salt chosen from among the ammonium and phosphonium salts having formulae (IV) and (V) respectively:

$$N(R')_4X \qquad (IV)$$

and

$$P(R')_4X \qquad (V)$$

wherein $(R')_4$ represents a homogeneous or heterogeneous hydrocarbyl radical group containing up to 20 carbon atoms, and wherein X is a halogen as herein-above defined.

The addition of an "onium", phosphonium (V) or quaternary ammonium (IV) salt, occurs according to a molar ratio of onium salt/cobalt hydrocarbonyl salt catalyst comprised between about 1:1 and 3:1. These values are to be considered as practical values that are not critical with respect to the effectiveness of the reaction.

Effective "onium" salts proved to be: 1-phenyl-1-ethyltrimethylammonium iodide, phenyltrimethylammonium bromide or iodide, tetra-n-butylammonium bromide, and benzyltrimethylammonium chloride, etc. Obviously, for the purposes of the reaction, it is also possible to use other "onium" salts or to use crown-ethers being, as is known to those skilled in the art, functional equivalents with regard to the reaction itself of the phosphonium and ammonium salts of the invention.

The reaction may also be conducted in the absence of "onium" salts, in which case however, the reaction will lead to results of a lower interest.

At the end of the reaction, the reaction product is separated according to known techniques.

The product obtained in the form of a salt, according to the nature of the biphasic system employed (type of the solvent and concentration of the base) may be found dissolved in the aqueous or organic phases, or else in the form of a precipitate.

In the first case, after separation of the aqueous phase from the organic phase containing the catalyst, which is recycled, one proceeds with the acidification (with HCl, $H_2SO_4$) and with the extraction by means of a solvent, etc.

In the second case, the organic phase, after separation from the recyclable aqueous phase, is washed with water and then treated as previously indicated.

In the third case, the precipitate is, filtered, etc. and the liquid phases are recycled. Obviously, the recycled liquid phases are restored to the initial state of the composition.

The process, according to an effective embodiment, is conducted in the following way.

In a reactor, provided with a stirrer, and with a temperature regulating system, are introduced, under a carbon mono-oxide atmospheric pressure: the organic solvent, the aqueous alkaline solution (NaOH), the "onium" salt and the catalyst or its precursor.

This reaction mixture is brought to the pre-established temperature and pressure, whereafter the 1-halogen-1-arylethane (II) is introduced and the mixture is then maintained under vigorous stirring at the same temperature till completion of the reaction (i.e. the stopping of the absorption of CO). Thereupon one proceeds with the separation of the product as herein-above described. The techniques used are essentially conventional techniques.

The process, due to the simple and mild operative conditions, appears to be particularly convenient. The invention will now be described in further detail by means of the following examples given, however, for merely illustrative purpose.

Examples 2, 5 and 10 have been conducted in the absence of "onium" salts.

Example 1

Into a 100 ml flask, fitted with a magnetic stirrer, a thermometer and a coolant, were introduced under a CO atmosphere:

. 25 ml of n-butyl alcohol,
. 50 ml of an aqueous 50% solution of KOH,
. 0.43 g of $Co_2(CO)_8$, and
. 0.7 g of phenyltrimethylammonium bromide

This mixture was thereupon brought to a temperature of 35°C and, after 15 minutes, 8.3 g of 1-bromo-1-phenylethane were added over a period of 5 hours. At the end of this addition the reaction mixture was kept under stirring for a further 2 hours.

Thereupon the organic phase was separated and washed with water. The washing water, added to the first aqueous phase, was acidified with HCl and then extracted by using ethyl ether. After evaporation of the ether, 3.7 g of alpha-phenyl-propionic or hydroatropic acid were obtained (yield: 55% with respect to the initial halide).

Example 2

Into the apparatus as described in example 1, and following the same procedures, were introduced:

. 25 ml of n-butyl alcohol,
. 50 ml of a 50% aqueous KOH solution,
. 0.45 g of $Co_2(CO)_8$

Thereupon the temperature of the mixture was brought to 35°C and, after about 15 minutes, 8.3 g of 1-bromo-1-phenylethane were added over a period of 5 hours. At the end of the addition, the reaction mixture was maintained under stirring for a further 2 hours.

By proceeding as described in Example 1, approximately 3.3 g of hydroatropic acid (yield: 49%), and 0.27 g of hydrocynnamic acid (yield; 4%) were obtained.

Example 3

Into the apertures as described in Example 1 and following the same procedures, were introduced:

. 25 ml of t.amyl alcohol,
. 25 ml of an aqueous 50% KOH solution,
. 0.46 g of $Co_2(CO)_8$, and
. 1 g of alpha-phenylethyltrimethylammonium iodide.

Thereupon the reaction mixture was brought to a temperature of 35°C and, after about 15 minutes, 4.1 g of 1-bromo-1-phenylethane were introduced. The reaction mixture was then subjected to stirring for 12 hours and, by proceeding as described in Example 1, 1.5 g of hydroatropic acid were obtained (yield: 45.1%).

Example 4

Into the apparatus as described in Example 1, and following the same procedures, were introduced:

. 25 ml of t.amyl alcohol,
. 25 ml of a 40% aqueous NaOH solution,
. 0.43 g of $Co_2(CO)_8$, and
. 1 g of alpha-phenylethyl-trimethylammonium iodide

The reaction mixture was then brought to a temperature of 35°C and, after about 15 minutes, 4.1 g of 1-bromo-1-phenylethane were introduced. The reaction mixture was then subjected to stirring for a period of 5 hours, whereafter, by proceeding as described in Example 1, approximately 1.85 g of hydroatropic acid (yield: 55.6%) and 0.07 g of hydrocynnamic acid (yield: 2.1%) were obtained.

Example 5

Into the apparatus as described in Example 1, and following the same procedures, were introduced:

. 25 ml of t.amyl alcohol,
. 25 ml of a 40% aqueous NaOH solution, and
. 0.43 g of $Co_2(CO)_8$

The temperature of the reaction mixture was thereupon brought to 35°C and, after about 15 minutes, 4.1 g of 1-bromo-1-phenylethane were introduced. The reaction mixture was then subjected to stirring for 5 hours, whereafter, by proceeding as described in Example 1, 1.43 g of hydroatropic acid approximately (yield: 43%) and 0.39 g of hydrocynnamic acid (yield: 11.7%) were obtained.

Example 6

Into the apparatus as described in Example 1, and following the same procedures, were introduced:

. 25 ml of n-butyl alcohol,
. 25 ml of a 50% aqueous KOH solution,
. 0.5 g of $NaCO(CO)_4$, and
. 0.7 g of phenyltrimethylammonium bromide

The temperature of the reaction mixture was then brought to 35°C and 4.1 g of 1-bromo-1-phenylethane were added. The reaction mass was then subjected to stirring for 4 hours and by proceeding as described in Example 1, 1.45 g of hydroatropic acid were obtained (yield: 43.6%).

Example 7

Into the apparatus as described in Example 1, and following the same procedures were introduced:

. 25 ml of 1-phenylethanol,
. 25 ml of a 50% aqueous KOH solution,
. 0.45 g of $Co_2(CO)_8$, and
. 1 g of alpha-phenylethyl-trimethylammonium iodide.

The reaction mixture was thereupon brought to a temperature of 35°C and, after 15 minutes, 4.1 g of 1-bromo-1-phenylethane were added. The mixture was then subjected to stirring for 7 hours, whereupon, by proceeding as described in Example 1, 1.3 g of hydroatropic acid were obtained (yield: 39.1%).

Example 8

Into the apparatus as described in Example 1, and following the same procedures, were introduced:

. 25 ml of n-propyl alcohol,
. 25 ml of a 50% aqueous KOH solution,
. 0.45 g of $Co_2(CO)_8$, and
. 0.7 g of phenyltrimethylammonium bromide

The temperature of the reaction mixture was then brought to 35°C, after 15 minutes, 4.1 g of 1-bromo-1-phenylethane, were added. The mixture was then subjected to stirring for 7 hours after which, by proceeding as described in Example 1, 1.1 g of hydroatropic acid were obtained (yield: 33.1%).

Example 9

Into the apparatus as described in Example 1, and following the same procedures, were introduced:

. 25 ml of t-butylmethylether,
. 25 ml of a 50% aqueous KOH solution,
. 0.42 g of $Co_2(CO)_8$, and
. 0.6 g of benzyltrimethylammonium chloride

The mixture was thereupon brought to a temperature of 35°C and, after about 15 minutes, 4 g of 1-bromo-1-phenylethane, were introduced. The reaction mass was then kept under stirring for 4 hours. At the end of this period the reaction mixture was filtered; the solid thus obtained was diluted with water, acidified with hydrochloric acid and extracted by using ethyl ether. After evaporation of the ether, 1.38 g of hydroatropic acid were obtained (yield: 42.5%) and 0.23 g of phenylacetic acid were produced from the ammonium salt.

Example 10

Into the apparatus as described in Example 1, and following the same procedures, were introduced:

. 25 ml of t-butylmethylether,
. 25 ml of a 50% aqueous KOH solution,
. 0.43 g of $Co_2(CO)_8$

This mixture was then brought to a temperature of 35°C and, after about 15 minutes, 4.1 g of 1-bromo-1-phenylethane were added. The mixture was then kept under stirring for 4 hours after which, by proceeding as described in Example 9, 1.65 g of hydroatropic acid were obtained (yield: 49.6%) and 0.25 g of hydrocynnamic acid (yield: 7.5%) were obtained.

Example 11

Into the apparatus as described in Example 1, and following the same procedures, were introduced:

. 25 ml of diphenylether,
. 25 ml of a 50% aqueous KOH solution,
. 0.43 g of $Co_2(CO)_8$, and
. 0.7 g of phenyltrimethylammonium bromide

This mixture was then brought to a temperature of 35°C and, after about 15 minutes, 4.1 g of 1-bromo-1-phenylethane were added. The mixture was then kept under stirring for 7 hours. By proceeding as described in Example 9, 1.3 g of hydroatropic acid (yield: 39.1%) and 0.65 g of hydrocynnamic acid (yield: 1.9%) were obtained.

Example 12

Into the apparatus as described in Example 1, and following the same procedures, were introduced:

. 25 ml of benzene,
. 25 ml of a 50% aqueous KOH solution,
. 0.43 g of $Co_2(CO)_8$, and
. 0.6 g of phenyltrimethylammonium bromide

The mixture was thereupon brought to a temperature of 35°C and, after about 15 minutes, it was additioned with 4.1 g of 1-bromo-1-phenylethane. The mixture was thereupon kept under stirring for 12 hours, whereafter, by proceeding as described in Example 9, 1.1 g of hydroatropic acid were obtained (yield: 33.1%).

Example 13

Into the apparatus as described in Example 1, and following the same procedures, were introduced:

. 25 ml of n-butyl alcohol,
. 25 ml of a 50% aqueous KOH solution,
. 0.48 g of $Co_2(CO)_8$, and
. 0.7 g of phenyltrimethylammonium bromide

The mixture was thereupon brought to a temperature of 35°C and, after about 15 minutes, was additioned with 5 g of 1-bromo-1-(p.chlorophenyl)-ethane over a period of 3 hours. At the end of this addition, the mixture was subjected to stirring for a further 2 hours. Then, by proceeding as described in Example 1, 2 g of alpha-(p.chlorophenyl)-propionic acid were obtained (yield: 47.7%).

Example 14

Into the apparatus as described in Example 1, and following the same procedures, were introduced:

. 25 ml of n-butyl alcohol,
. 25 ml of a 50% aqueous KOH solution,
. 0.43 g of $Co_2(CO)_8$, and
. 0.7 g of phenyltrimethylammonium bromide

The mixture was thereupon brought to a temperature of 35°C and, after about 15 minutes, 5.1 g of 1-bromo-1-(m.chlorophenyl)-ethane was added to the mixture, over a period of 3 hours. The mixture was then kept under stirring for 2 hours and, by proceeding as described in Example 1, 1.71 g of alpha-(m.chlorophenyl)-propionic acid were obtained (yield: 39.9%).

Example 15

Into the apparatus as described in Example 1, and following the same procedures, were introduced:

. 25 ml of n-butyl alcohol,
. 25 ml of a 50% aqueous KOH solution,
. 0.47 g of $Co_2(CO)_8$, and
. 0.7 g of phenyltrimethylammonium bromide

The mixture was then brought to a temperature of 35°C, and after about 15 minutes, 5.4 g of 1-bromo-1-(p.isobutylphenyl)-ethane were added over a period of 4 hours. The mixture was then kept under stirring for 2 hours, after which, by proceeding as described in Example 1, 2.5 g of alpha-(p.isobutylphenyl)-propionic acid were obtained (yield: 54.2%).

Example 16

Into the apparatus as described in Example 1, and following the same procedures, were introduced:

. 25 ml of n-butyl alcohol,
. 25 ml of a 50% aqueous KOH solution,
. 0.45 g of $Co_2(CO)_8$, and
. 0.7 g of phenyltrimethylammonium bromide

This mixture was brought to a temperature of 35°C and, after 15 minutes, 5 g of 1-chloro-1-(6'-methoxy-2'-naphthyl)-ethane were added over a period of 6 hours. The mixture was then kept under stirring for 2 hours and, by proceeding as described in Example 1, 1.82 g of alpha-(6'methoxy-2'-naphthyl)-propionic acid were obtained (yield: 34.9%).

Example 17

Into a flask fitted with a thermometer, a coolant and a dropping funnel, were introduced, under a CO atmosphere:

. 25 ml of t.amyl alcohol,
. 25 ml of a 20% aqueous NaOH solution,
. 0.15 g of $Co_2(CO)_8$, and
. 0.3 g of benzyltrimethylammonium chloride

The mixture was thereupon brought to a temperature of 45°C and the CO pressure to 2 atmospheres. After about 15 minutes, 4.1 g of 1-bromo-1-phenylethane were introduced over a period of 5 hours. The mixture was then kept for 10 hours under stirring at the same pressure, whereafter, by proceeding as in Example 1, 1.8 g of acid product were obtained. The recovered product, by gas-chromatography analysis proved to be a mixture of hydroatropic acid and 3-methyl-3-phenylpyruvic acid in a ratio of about 1:1. The latter compound was identified by the mass spectrum of the product:

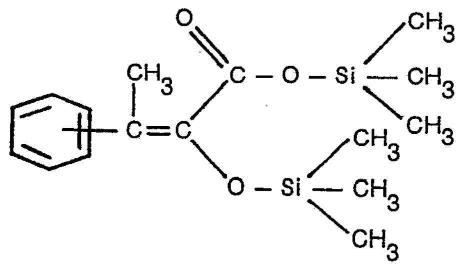

obtained by treatment at 60°C, in an anhydrous medium, of the acid with a mixture consisting of:

. 10 parts of pyridine,
. 0.5 parts of trimethylchloroxylane and
. 2 parts of N, O Bis-trimethylsilylacetamide

Example 18

Into the apparatus as described in Example 1, and following the same procedures, were introduced into the reactor:

. 25 ml of n-butyl alcohol,
. 25 ml of a 50% aqueous KOH solution,
. 0.45 g of $Co_2(CO)_8$, and
. 0.7 g of phenyltrimethylammonium bromide

The mixture was then brought to a temperature of 45°C and, after about 15 minutes, 3.3 g of 1-chloro-1-phenylethane were added. The mixture was then kept for 20 hours under stirring, after which by proceeding as described in Example 1, 2.04 g of hydroatropic acid (yield: 57.9%) and 0.04 g of hydrocynnamic acid (yield: 1.1%) were obtained.

Example 19

Into the apparatus as described in Example 17, and following the same procedures, were introduced:

. 25 ml of t-amyl alcohol,
. 25 ml of a 20% aqueous NaOH solution,
. 0.30 g of $Co_2(CO)_8$, and
. 0.6 g of phenylethyl-trimethylammonium iodide

This mixture was thereupon brought to a temperature of 35°C and the CO pressure to 2 atmospheres. After about 15 minutes, 4.1 g of 1-bromo-1-phenylethane were added, over a period of 3 hours. The reaction mixture was then kept under stirring for 8 hours at the same pressure and, by proceeding as described in Example 1, 2.2 g of acid product were obtained.

The recovered product, by gas-chromatography analysis proved to consist of:

. hydroatropic acid 15% (10% yield with respect to the starting bromide),
. hydrocynnamic acid 2% (1.3% yield with respect to the starting bromide),
. 3-methyl-3-phenylpyruvic acid 83% (55% yield with respect to the starting bromide).

Example 20

Into the apparatus as described in Example 1, and following the same procedures, were introduced:

. 25 ml of t-amyl alcohol,
. 25 ml of a 50% aqueous KOH solution,
. 0.45 g of $Co_2(CO)_8$, and
. 0.7 g of phenyltrimethylammonium bromide

The temperature was kept at 15°C and, after 15 minutes about, 3 g of 1-chloro-1-(2-furyl)ethane were added over a period of 4 hours. At the end of the addition the mixture was then kept under stirring during 2 hours.

Then, by proceeding as described in Example 1, 0.7 g of $\alpha$-(2-furyl) propionic acid were obtained (yield: 21.7%).

**Claims**

1. A process for the preparation of alkaline salts of carboxylic acids having the formula (I):

$$\begin{array}{l} Ar\text{—}CH\text{—}(CO)_nOH \\ \qquad\;\; | \\ \qquad\; CH_3 \end{array} \qquad (I)$$

wherein:

— Ar represents either an aromatic or a heteroaromatic group containing one ring or several rings linked together, and having overall up to 20 carbon atoms, preferably being chosen from amongst phenyl, naphthyl, diphenyl and thienyl groups, also substituted with groups inert under reaction conditions, preferably chosen from amongst alkyl, cycloalcyl and aryl groups optionally substituted, halogen, alkoxyl, phenoxyl and ketone groups,
— and n is an integer equal to 1 or 2,

characterized in that a 1-halogen-1-arylethane of formula (II):

$$\begin{array}{l} Ar\text{—}CH\text{—}X \\ \qquad\;\; | \\ \qquad\; CH_3 \end{array} \qquad (II)$$

wherein:

— Ar has the above meaning, and
— X is Cl or Br,

is made to react with carbon monoxide and an alkaline hydroxide, in the presence of a cobalt hydrocarbonyl salt catalyst or at least one of its precursors, at a temperature comprised between about 20°C and 70°C and at a pressure comprised between about 1 and 10 atmospheres in a biphasic aqueous/organic liquid system consisting of:

a) an aqueous phase containing the alkaline hydroxide, and

b) an organic phase consisting of the initial halide of formula (II) dissolved in the organic solvent substantially unmixable with the aqueous alkaline phase, in the presence of an "onium" salt and a catalyst consisting of a cobalt hydrocarbonyl salt or at least one of its precursors.

2. A process according to claim 1, characterized in that the organic solvent is chosen from amongst:

. aliphatic and cycloaliphatic linear or branched alcohols having 3 to 10 carbon atoms, optionally aryl substituted;
. aromatic and aliphatic ethers;
. aromatic hydrocarbons.

3. A process according to claim 2, characterized in that the organic solvent is chosen from amongst the compounds belonging to the group comprising benzene, diphenylether, t-butyl-methyl-ether, n-propyl alcohol, isopropyl, alcohol, n-butyl alcohol, sec.-butyl alcohol, neopentyl alcohol, n-amyl alcohol, t-amyl alcohol, cyclohexanol and 1-phenylethanol.

4. A process according to claim 1, characterized in that the alkaline hydroxide is chosen from amongst sodium potassium or lithium hydroxides, preferably from between sodium and potassium hydroxides.

5. A process according to claim 1, characterized in that the cobalt hydrocarbonyl salt catalyst has the formula (III):

$$Me^{n+}\{Co(CO)_4\}_n \qquad (III)$$

wherein Me represents a metal of valence n, preferably chosen from amongst Na, K, Li, Co, Mn and Fe.

6. A process according to claim 5, characterized in that the catalyst consists of a cobalt hydrocarbonyl salt chosen from amongst sodium, cobalt, iron and manganese salts.

7. A process according to claim 5, characterized in that the cobalt hydrocarbonyl salt catalyst is obtained from a cobalt salt, preferably chosen from amongst chloride bromide and sulphate salts, from a iron-manganese powder alloy with 80% of Mn content, or from sulphurated promoters, preferably chosen from amongst the alkaline sulphides and thiosulphates, in an alcohol medium chosen from between methyl and ethyl alcohol, by reaction with CO under a pressure comprised between 1 and 20 atmospheres and at a temperature comprised between about 10°C and 80°C, preferably comprised between about 25°C and 35°C.

8. A process according to claim 5, characterized in that the cobalt hydrocarbonyl alkaline salt catalyst is prepared *"in situ"* from $Co_2(CO)_8$ in the alkaline medium forming the reaction medium.

9. A process according to claim 1, characterized in that the molar ratio between the 1-halogen-1-arylethane (II) halide and the cobalt hydrocarbonyl salt catalyst is comprised between about 10:1 and 150:1.

10. A process according to claim 1, characterized in that the concentration of the alkaline base, consisting of sodium or potassium hydroxides, in the aqueous phase, is comprised between about 20% and 50% by weight.

11. A process according to claim 1, characterized in that the 1-halogen-1-arylethane (II) is selected from the group formed by 1-bromo-1-phenylethane, 1-chloro-1-(6′-methoxy-2′-naphthyl)-ethane, 1-bromo-1-(p.chlorophenyl)-ethane, 1-bromo-1-(p.isobutyl-phenyl)-ethane, 1-bromo-1-(m.chlorophenyl)-ethane, 1-chloro-1-phenylethane.

12. A process according to claim 1, characterized in that the "onium" salt is chosen from among the ammonium and phosphonium salts of formulae:

$$N(R')_4X \qquad (IV)$$

and

$$P(R')_4X \qquad (V)$$

wherein:

— $(R')_4$ represents either a homogeneous or heterogeneous hydrocarbyl radical containing up to 20 carbon atoms, and
— X is a halogen consisting of Cl or Br, the "onium" salt being preferably chosen from the group formed by 1-phenyl-ethyltrimethylammonium iodide, tetra-n-butylammonium bromide, benzyltrimethylammonium chloride, phenyltrimethylammonium bromide or iodide.

13. A process according to claim 11, characterized in that the "onium" salt is used in a molar ratio comprised between about 1:1 and 3:1 with respect to the moles of the cobalt hydrocarbonyl salt catalyst.

14. A process according to claim 1, characterized in that, to obtain the compounds of formula (I) where n=2, pressures≥2 atmospheres are used.

**Patentansprüche**

1. Verfahren zur Herstellung von Alkalisalzen von Carbonsäuren mit der Formel (I):

$$\underset{\displaystyle CH_3}{\overset{}{Ar—\underset{|}{CH}—(CO)_nOH}} \qquad (I)$$

in welcher:

— Ar entweder für eine aromatische oder eine heteroaromatische Gruppe mit einem Ring oder mehreren miteinander verbundenen Ringen, die insgesamt bis zu 20 Kohlenstoffatome aufweisen, steht und vorzugsweise ausgewählt wurde von: Phenyl-, Naphthyl-, Diphenyl- und Thienylgruppen, gegebenenfalls auch mit unter den Reaktionsbedingungen inerten Gruppen substituiert, welche vorzugsweise ausgewählt wurden von: Alkyl-, Cycloalkyl- und Arylgruppen, die gegenbenenfalls substituiert sind, Halogen-, Alkoxy, Phenyloxy- und Keton-Gruppen
— und n 1 oder 2 bedeutet,

dadurch gekennzeichnet, daß man ein 1-Halogen-1-arylethan der Formel (II):

$$\underset{\displaystyle CH_3}{\underset{|}{Ar—CH—X}} \qquad (II)$$

in welcher

— Ar die oben angegebene Bedeutung und
— X=Cl oder Br ist,

mit Kohlenmonoxid und einem Alkalihydroxid in Anwesenheit eines Kobalt-Hydrocarbonyl-Salz-Katalysators oder wenigstens einem von dessen Vorläufern bei einer Temperatur zwischen etwa 20°C und 70°C und einem Druck zwischen etwa 1 und 10 Atmosphären in einem zweiphasigen wässrig/organischen flüssigen System umsetzt, welches besteht aus:

a) einer wässrigen Phase, die das Alkalihydroxid enthält und

b) einer organischen Phase, welche aus dem ursprünglichen Halogenid der Formel (II), das in einem mit der wässigen alkalischen Phase praktisch unmischbaren organischen Lösungsmittel gelöst ist, besteht, in Gegenwart eines "Onium"-Salzes und einem aus Kobalt-Hydrocarbonyl-Salz oder wenigstens einem von dessen Vorläufern bestehenden Katalysators.

2. Verfahren nach Anspruch 1, dadurch gekennzeichnet, daß das organische Lösungsmittel ausgewählt wurde von:

aliphatischen und cycloaliphatischen, linearen oder verzweigten Alkoholen mit 3 bis 10 Kohlenstoffatomen, welche gegebenenfalls Aryl-substituiert sing;
aromatischen und aliphatischen Ethern;
aromatischen Kohlenwasserstoffen.

3. Verfahren nach Anspruch 2, dadurch gekennzeichnet, daß das organische Lösungsmittel ausgewählt wurde von Verbindungen, die zu der Gruppe gehören, welche umfaßt:

Benzol, Diphenylether, t-Butyl-methyl-ether, n-Propylalkohol, Isopropylalkohol, n-Butylalkohol, sec.-Butylalkohol, Neopentylalkohol, n-Amylalkohol, t-Amylalkohol, Cyclohexanol und 1-Phenylethanol.

4. Verfahren nach Anspruch 1, dadurch gekennzeichnet, daß das Alkalihydroxid ausgewählt wurde von: Natrium-, Kalium- oder Lithiumhydroxid, vorzugsweise von Natrium- und Kaliumhydroxid.

5. Verfahren nach Anspruch 1, dadurch gekennzeichnet, daß der Kobalt-Hydrocarbonyl-Salz-Katalysator die Formel (III):

$$Me^{n+}\{CO(CO)_4\}_n \qquad (III)$$

besitzt, worin Me für ein Metal der Wertigkeit n steht und vorzugsweise ausgewählt ist von Na, K, Li, Co, Mn und Fe.

6. Verfahren nach Anspruch 5, dadurch gekennzeichnet, daß der Katalysator aus einem Kobalt-Hydrocarbonyl-Salz besteht, wobei dieses von Natrium-, Kobalt, Eisen- und Mangansalzen ausgewählt ist.

7. Verfahren nach Anspruch 5, dadurch gekennzeichnet, daß der Kobalt-Hydrocarbonyl-Salz-Katalysator erhalten worden ist aus einem Kobaltsalz, vorzugsweise ausgewählt von Chlorid-, Bromid- und Sulfatsalzen, einem Eisen-Mangan-Legierungs-Pulver mit 80% Mangangehalt, bzw. Schwefel-Promoteren, vorzugsweise ausgewählt von Alkalisulfiden und -Thiosulfaten, in einem Alkoholmedium, ausgewählt von Methylalkohol und Ethylalkohol, durch Umsetzung mit CO bei einem Druck zwischen 1 und 20 Atmosphären und einer Temperatur zwischen etwa 10°C und 80°C, vorzugsweise zwischen etwa 25°C und 35°C.

8. Verfahren nach Anspruch 5, dadurch gekennzeichnet, daß der Kobalt-Hydrocarbonyl-Alkalisalz-Katalysator aus $Co_2(CO)_8$, in dem das Reaktionsmedium bildende alkalische Medium in situ hergestellt worden ist.

9. Verfahren nach Anspruch 1, dadurch gekennzeichnet, daß das Mol-Verhältnis zwischen dem 1-Halogen-1-arylethan (II) und dem Kobalt-Hydrocarbonyl-Salz-Katalysator zwischen etwa 10:1 und 150:1 beträgt.

10. Verfahren nach Anspruch 1, dadurch gekennzeichnet, daß die Konzentration der Alkalibase, bestehend aus Natrium- oder Kaliumhydroxid, in der wässrigen Phase zwischen etwa 20 Gew.-% und 50 Gew.-% beträgt.

11. Verfahren nach Anspruch 1, dadurch gekennzeichnet, daß das 1-Halogen-1-arylethan (II) ausgewählt wurde aus der Gruppe: 1-Brom-1-phenylethan, 1-Chlor-1-(6′-methoxy-2′-naphthyl)-ethan, 1-Brom-1-(p-chlorphenyl)-ethan, 1-Brom-1-(p-isobutyl-phenyl)-ethan, 1-Brom-1-(m-chlorphenyl)-ethan, 1-Chlor-1-phenylethan.

12. Verfahren nach Anspruch 1, dadurch gekennzeichnet, daß das "Onium"-Salz ausgewählt ist von den Ammonium- und Phosphonium-Salzen der Formeln:

$N(R')_4X$ (IV)

und

$P(R')_4X$ (V)

in welchen:

— $(R')_4$ für eine homogene oder heterogene Hydrocarbylgruppe mit bis zu 20 Kohlenstoffatomen steht und
— X ein Halogen, nämlich Cl oder Br, ist, wobei das "Onium"-Salz vorzugsweise ausgewählt ist von der Gruppe: 1-Phenyl-ethyl-trimethylammonium-jodid, Tetra-n-butylammonium-bromid, Benzyltrimethylammonium-chlorid, Phenyl-trimethalammonium-bromid oder -jodid.

13. Verfahren nach Anspruch 11, dadurch gekennzeichnet, daß das "Onium"-Salz in einem Mol-Verhältnis zwischen etwa 1:1 und 3:1, in bezug auf die Mol-Menge des Kobalt-Hydrocarbonyl-Salz-Katalysators verwendet wird.

14. Verfahren nach Anspruch 1, dadurch gekennzeichnet, daß zur Herstellung der Verbindungen nach Formel (I), worin n=2 ist, Drücke≥2 Atmosphären verwendet werden.

**Revendications**

1. Procédé de préparation de sels alcalins d'acides carboxyliques ayant la formule (I):

$$Ar—CH(CH_3)—(CO)_nOH \qquad (I)$$

dans laquelle:

Ar représente soit un groupe aromatique, soit un groupe hétéroaromatique contenant un cycle ou plusieurs cycles reliés ensemble et possédant ensemble jusqu'à 20 atomes de carbone, choisis de préférence parmi les groupes phényl, naphtyl, diphényl ou thiényl, également substitués par des groupes inertes dans les conditions de la réaction, choisis de préférence parmi les groupes alkyl, cycloalkyl et aryl éventuellement substitués et les groupes halogène, alkoxyl, phénoxyl et cétone,
n est un entier égal à 1 ou 2,

caractérisé en ce que l'on fait réagir un 1-halogèno-1-aryléthane de formule (II)

$$Ar—CH(CH_3)—X \qquad (II)$$

dans laquelle:

Ar a la signification ci-dessus, et
X représente Cl ou Br,

avec le monoxyde de carbone et un hydroxyde alcalin en présence d'un catalyseur à base de sels d'hydrocarbonyl cobalt ou au moins l'un de ses précurseurs à une température comprise entre environ 20°C et 70°C et à une température comprise entre environ 1 et 10 atmosphères dans un système liquide biphasique organo/aqueux constitué de:
a)une phase aqueuse contenant l'hydroxyde alcalin; et
b) une phase organique constituée de l'halogénure initial de formule (II) dissous dans un solvant organique sensiblement non miscible avec la phase alcaline aqueuse,

en présence d'un sel de "onium" et d'un catalyseur constitué d'un sel d'hydrocarbonyl cobalt ou au moins l'un de ses précurseurs.

2. Procédé selon la revendication 1, caractérisé en ce que le solvant organique est choisi parmi:

— les alcools, linéaires ou ramifiés, aliphatiques et cycloaliphatiques possédant de 3 à 10 atomes de carbone, éventuellement aryles substitués;
— les éthers aromatiques et aliphatiques;
— les hydrocarbures aromatiques.

3. Procédé selon la revendication 2, caractérisé en ce que le solvant organique est choisi parmi les composés appartenant au groupe comprenant benzène, diphényléther, t-butyl-méthyl-éther, alcool n-propylique, alcool isopropylique, alcool n-butylique, alcool butylique secondaire, alcool néo-penthylique, alcool n-amylique, alcool t-amylique, cyclo-héxanol et 1-phényléthanol.

4. Procédé selon la revendication 1, caractérisé en ce que l'hydroxyde alcalin est choisi parmi les hydroxydes de sodium, potassium ou lithium, de préférence parmi les hydroxydes de sodium et de potassium.

5. Procédé selon la revendication 1, caractérisé en ce que le catalyseur à base de sels d'hydrocarbonyl cobalt possède la formule (III):

$$Me^{n+}[Co(CO)_4]_n \qquad (III)$$

dans laquelle:

Me représente un métal de valence n, choisi de préférence parmi Na, K, Li, Co, Mn et Fe.

6. Procédé selon la revendication 5, caractérisé en ce que le catalyseur consiste en un sel d'hydrocarbonyl cobalt choisi parmi les sels de sodium, cobalt, fer et manganèse.

7. Procédé selon la revendication 5, caractérisé en ce que le catalyseur à base de sels d'hydrocarbonyl cobalt est obtenu à partir d'un sel de cobalt choisi de préférence parmi les chlorure, bromure et sulfate, à partir d'une poudre d'alliage de manganèse ayant une teneur en manganèse de 80% ou à partir de promoteurs sulfurés choisis, de préférence, parmi les sulfures et thiosulfates alcalins, dans un milieu alcoolique choisi entre l'alcool méthylique et l'alcool éthylique, par réaction avec CO à une pression comprise entre 1 et 20 atmosphères et à une température comprise entre environ 10°C et 80°C, de préférence comprise entre environ 25°C et 35°C.

8. Procédé selon la revendication 5, caractérisé en ce que le catalyseur à base de sels alcalins d'hydrocarbonyl cobalt est préparé in situ à partir de $Co_2(CO)_8$ dans le milieu alcalin formant le milieu réactionnel.

9. Procédé selon la revendication 1, caractérisé en ce que le rapport molaire entre l'halogénure de 1-halogèno-1-aryléthane (II) et le catalyseur à base de sels d'hydrocarbonyl cobalt est compris entre environ 10/1 et 150/1.

10. Procédé selon la revendication 1, caractérisé en ce que la concentration de la base alcaline constituée des hydroxydes de sodium ou de potassium en phase aqueuse est comprise entre environ 20% et 50% en poids.

11. Procédé selon la revendication 1, caractérisé en ce que le 1-halogèno-1-aryléthane (II) est choisi dans le groupe constitué par 1-bromo-1-phényléthane, 1-chloro-1-(6'-méthoxy-2'-naphtyl)-éthane, 1-bromo-1-(p.chlorophényl)-éthane, 1-bromo-1-(p.isobutylphényl)-éthane, 1-bromo-1-(m-chlorophényl)-éthane, 1-chloro-1-phényléthane.

12. Procédé selon la revendication 1, caractérisé en ce que le sel de "onium" est choisi parmi les sels d'ammonium et de phosphonium de formule:

$$N(R')_4X \qquad (IV)$$

et

$$P(R')_4X \qquad (V)$$

dans lesquelles:

$(R')_4$ représente soit un radical hydrocarbure homogène, soit un radical hydrocarbure hétérogène contenant jusqu'à 20 atomes de carbone; et

X représente un halogène constitué de Br ou de Cr, le sel de "onium" étant de préférence choisi dans le groupe constitué par iodures de 1-phényl-éthyltriméthylammonium, bromure de tétra-n-butylammonium, chlorure de benzyltriméthylammonium, bromure ou iodure de phényltriméthylammonium.

13. Procédé selon la revendication 11, caractérisé en ce que le sel de "onium" est utilisé dans un rapport molaire compris entre environ 1/1 et 3/1 par rapport aux moles de catalyseur à base de sels d'hydrocarbonyl cobalt.

14. Procédé selon la revendication 1, caractérisé en ce que, pour obtenir les composés de formule (I) duns lesquels n=2, on utilise des pressions≥à 2 atomsphères.